# EUROPEAN PATENT APPLICATION

(11) **EP 1 466 896 A1**
(43) Date of publication of application: **13.10.2004**
(21) Application number: 02792065.1
(22) Date of filing: 27.12.2002
(51) Int. Cl.: C07C 311/18, C07C 311/41, C07C 303/36, C07C 303/40, C07C 303/44

(54) **PROCESS FOR PRODUCING CRYSTAL OF BENZENESULFONAMIDE DERIVATIVE, AND NOVEL CRYSTAL OF INTERMEDIATE THEREFOR AND PROCESS FOR PRODUCING THE SAME**

(30) Priority: 28.12.2001 JP 2001401270; 13.03.2002 JP 2002069171
(71) Applicant: Ajinomoto Co., Inc., Tokyo 104-8315 (JP)
(72) Inventor: KURODA, S., AJINOMOTO CO., INC. Amino Sc. Lab., Kawasaki-shi, Kanagawa 210-8681 (JP); ONISHI, T., AJINOMOTO CO., INC. Amino Sc. Lab., Kawasaki-shi, Kanagawa 210-8681 (JP); HIROSE, N., AJINOMOTO CO., INC. Amino Sc. Lab., Kawasaki-shi, Kanagawa 210-8681 (JP); HAMADA, Takayuki c/o Ajinomoto Co.,Inc. Amino, Kawasaki-shi, Kanagawa 210-8681 (JP)
(74) Representative: Nicholls, Kathryn Margaret
(86) International application number: PCT/JP2002/013849
(87) International publication number: WO 2003/057665

(57) **Abstract**

The present invention relates to a method of producing a highly pure crystal, which includes crystallization of a benzenesulfonamide derivative of the following formula (1) using a polar solvent as a good solvent (e.g., alcohol or a mixed solvent of alcohol and water) and water as a poor solvent, and a novel crystal of a nitrobenzenesulfonamide derivative of the following formula (2), which is an intermediate for the derivative, and a production method thereof:

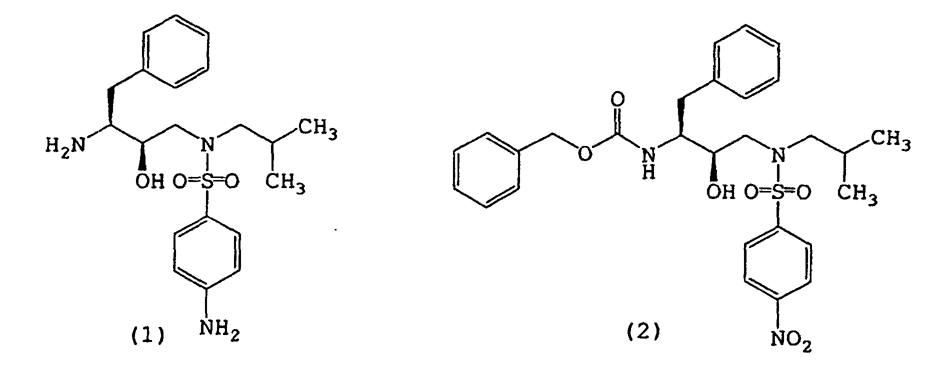

## Description

### Technical Field

The present invention relates to a production method of a crystal of (2R,3S)-N-(3-amino-2-hydroxy-4-phenylbutyl)-N-isobutyl-4-aminobenzenesulfonamide, and a novel crystal of an intermediate therefor and a production method thereof.

### Background Art

(2R,3S)-N-(3-Amino-2-hydroxy-4-phenylbutyl)-N-isobutyl-4-aminobenzenesulfonamide represented by the formula (1) 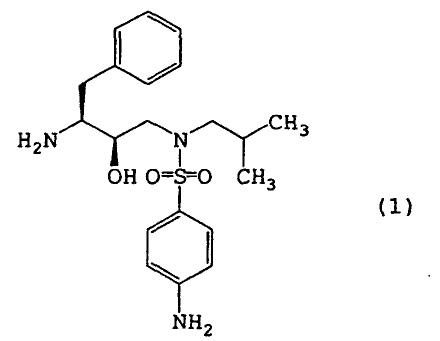 (hereinafter to be also referred to as benzenesulfonamide derivative (1)) and a salt thereof are compounds useful as intermediates for pharmaceutical compounds such as HIV protease inhibitor and the like (e.g., WO96/28418 (US Patent No. 6,140,505)).

As a production method of benzenesulfonamide derivative (1), for example, a method comprising hydrogenizing (2R,3S)-N-(3-benzyloxycarbonylamino-2-hydroxy-4-phenylbutyl)-N-isobutyl-4-nitrobenzenesulfonamide represented by the formula (2) 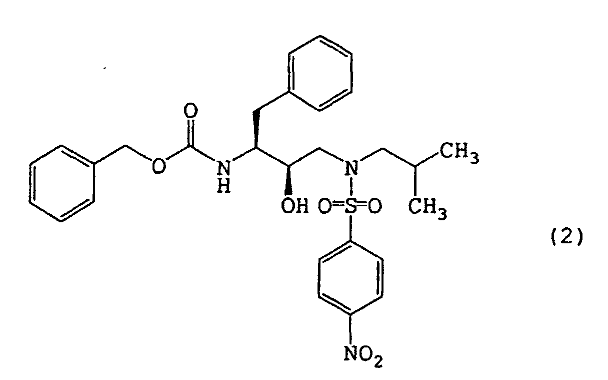 (hereinafter to be also referred to as nitrobenzenesulfonamide derivative (2)) dissolved in ethyl acetate, with a palladium catalyst, thereby to simultaneously conduct elimination (deprotection) of an amino-protecting group (benzyloxycarbonyl) and catalytic reduction of nitro group (WO96/28418 (US Patent No. 6,140,505), Example 21). In the Example, a reaction solvent is only evaporated after the completion of the reaction, with no purification step, wherein benzenesulfonamide derivative (1) was obtained only as a residue. Thus, there is no disclosure of a purification method of benzenesulfonamide derivative (1) and no report has documented that the derivative was obtained as a crystal.

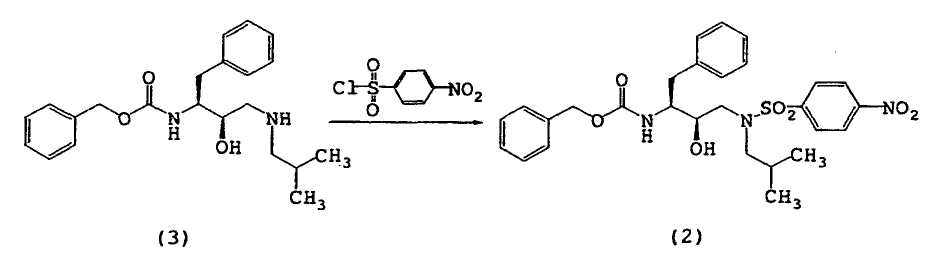

In addition, it is known that nitrobenzenesulfonamide derivative (2), which is an intermediate for benzenesulfonamide derivative (1), can be produced by reacting (2R,3S)-3-benzyloxycarbonylamino-2-hydroxy-1-(N-isobutylamino)-4-phenylbutane represented by the formula (3) (hereinafter to be also referred to as compound (3)) with p-nitrobenzenesulfonyl chloride as shown in the above-mentioned scheme (WO96/28418 (US Patent No. 6,140,505), Example 21). According to this method, ethyl acetate is added after the completion of the reaction, the mixture is washed with 5% citric acid, saturated sodium hydrogen carbonate and saturated sodium chloride aqueous solution, dried and concentrated, and the concentrate is recrystallized from ethyl acetate/hexane, whereby nitrobenzenesulfonamide derivative (2) is isolated and purified.

In this process, nitrobenzenesulfonamide derivative (2) dissolved in an organic solvent is once concentrated, and then dissolved in a different solvent. When practiced industrially, therefore, this method is hardly preferable from the aspects of efficiency, cost and the like. While the above-mentioned reference does not provide details of the drying method, for synthesis of a compound at a laboratory level, drying agents such as sodium sulfate, magnesium sulfate and the like are used for dehydration drying of an organic solvent and a method comprising adding these drying agents to the organic solvent and the like, and after stirring, removing the drying agents by filtering is generally employed. In addition, since the cost does not become a serious problem in the synthesis at a laboratory level, reaction solvents are often subjected to such dehydrating drying process even when dehydration drying is not necessarily required. In contrast, in an industrial process where production cost is an important problem, the dehydration drying process is generally omitted unless a problem occurs, such as a side reaction caused by water and the like. Even when dehydration drying is conducted, the above-mentioned method to be performed at a laboratory level is hardly suitable for industrial practice because it requires substantial costs for the purchase of drying agents, filter facility and the like, and increases the number of steps.

From the foregoing, an object of the present invention is to provide 1) an industrially useful production method of a crystal of benzenesulfonamide derivative (1), and 2) an industrially useful novel crystal of nitrobenzenesulfonamide derivative (2) and an industrially useful production method thereof.

### Disclosure of the Invention

The present inventors have conducted intensive studies in an attempt to develop a method (industrial production method) for producing benzenesulfonamide derivative (1) and nitrobenzenesulfonamide derivative (2) even on an industrial scale.

The benzenesulfonamide derivative (1) can be obtained by reacting compound (3) with p-nitrobenzenesulfonyl chloride to give nitrobenzenesulfonamide derivative (2), and subjecting this to catalytic reduction. When this method is performed on an industrial scale, it is considered to be a general practice also from an economical aspect to subject, after the completion of the reaction of compound (3) with p-nitrobenzenesulfonyl chloride, a reaction solution to a catalytic reduction step without subjecting the reaction solution (after concentration or evaporation of solvent as necessary) to an isolation and purification step. However, the present inventors have found that this method is associated with problems in that the catalytic reduction reaction (hydrogenation) proceeds markedly poorly, thereby causing an increased amount of catalytic reduction catalysts to be added, a longer reaction time, a lower yield and the like. The present inventors have further assumed that some substance is inhibiting this catalytic reduction reaction, and considered extracting nitrobenzenesulfonamide derivative (2) in the form of a crystal from the reaction solvent, and subjecting the crystal to a catalytic reduction step.

As a method for crystallizing nitrobenzenesulfonamide derivative (2) contained in a reaction solution on an industrial scale, cooling crystallization is considered to be most preferable. When, for example, a poor solvent is added in a large amount and crystallization is performed at a lower solubility of a solute, however, the cost necessary for the purchase of the solvent increases and the purification effect decreases. While concentration crystallization may be performed but generation of impurity due to heating is worried.

While the present inventors have extracted nitrobenzenesulfonamide derivative (2) as a crystal from a reaction solvent by these crystallization methods and subjected the crystal to a catalytic reduction step, the above-mentioned problems could not be solved.

The present inventors have conducted further studies and obtained the following findings.
[1] p-Nitrobenzenesulfonyl chloride, which is a starting material, inhibits the catalytic reduction reaction.
[2] The reaction of compound (3) with p-nitrobenzenesulfonyl chloride is carried out in the presence of a base, and a salt is produced as a by-product. To remove the salt, water is added and an extraction process is conducted. During the process, the majority of p-nitrobenzenesulfonyl chloride reacts with water to give p-nitrobenzenesulfonic acid but a part of p-nitrobenzenesulfonyl chloride remains.
[3] Crystals obtained by cooling crystallization (hereinafter to be referred to as crystal B) of a reaction solution (organic layer obtained by the extraction process of the above-mentioned [2]) containing nitrobenzenesulfonamide derivative (2) show poor filtration property and contain a residue of p-nitrobenzenesulfonyl chloride therein.
[4] Crystals obtained by dehydration drying and then crystallization (industrially, cooling crystallization is preferable) of a reaction solution (organic layer obtained by the extraction process of the above-mentioned [2]) containing nitrobenzenesulfonamide derivative (2) (hereinafter to be referred to as crystal A) show, unlike crystal B, superior filtration property and p-nitrobenzenesulfonyl chloride therein is removed.
[5] When a reaction solution (organic layer obtained by the extraction process of the above-mentioned [2]) containing nitrobenzenesulfonamide derivative (2) is subjected to crystallization at a comparatively high temperature (e.g., not less than 30°C) without dehydration drying, crystal A is obtained.
[6] The moisture contained in a reaction solution (organic layer obtained by the extraction process of the above-mentioned [2]) containing nitrobenzenesulfonamide derivative (2) mainly gets mixed in during the extraction process of the above-mentioned [2]. When hexane or heptane is added to the organic layer, an aqueous layer is separated, and the organic layer can be easily subjected to a dehydration drying step. Since hexane and heptane are poor solvents to nitrobenzenesulfonamide derivative (2), by subjecting the organic layer after removing aqueous layer to a crystallization (preferably cooling crystallization) step as it is, crystal A superior in filterability can be obtained. The dehydration drying can be also performed by washing the reaction solution with brine. In this case, a poor solvent such as hexane, heptane and the like needs to be added after dehydration drying to perform crystallization.

Moreover, the present inventors have found that benzenesulfonamide derivative (1) can be obtained as a highly pure crystal, from which impurities have been efficiently removed, by crystallization using a polar solvent as a good solvent and water as a poor solvent.

The present inventors have completed the present invention based on the above findings. Accordingly, the present invention includes the following.
1. A crystal of (2R,3S)-N-(3-benzyloxycarbonylamino-2-hydroxy-4-phenylbutyl)-N-isobutyl-4-nitrobenzenesulfonamide (nitrobenzenesulfonamide derivative (2)) represented by the formula (2) 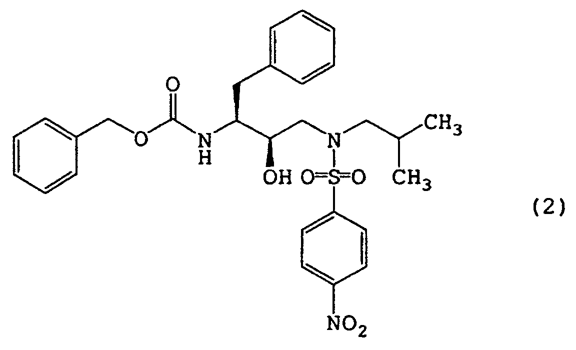 which has diffraction peaks at angles 2θ of 6.8°, 14.2° and 20.9° in a powder X-ray diffraction analysis.
2. A production method of a crystal of nitrobenzenesulfonamide derivative (2), which comprises subjecting a reaction mixture obtained by reacting (2R,3S)-3-benzyloxycarbonylamino-2-hydroxy-1-(N-isobutylamino)-4-phenylbutane with p-nitrobenzenesulfonyl chloride successively to step (i), step (ii) and step (iii), or successively to step (i), step (ii') and step (iii):
   (i) a step of adding water to carry out partitioning,
   (ii) a step of adding either or both of heptane and hexane to an organic layer obtained in the previous step to carry out partitioning,
   (ii') a step of washing the organic layer obtained in the previous step with brine, and then adding either or both of heptane and hexane thereto, and
   (iii) a step of cooling the organic layer obtained in the previous step.
3. The production method of the above-mentioned 2, which comprises washing the organic layer obtained in step (i) with an aqueous sodium hydrogen carbonate solution and then subjecting the layer to step (ii) or step (ii').
4. The production method of the above-mentioned 2, wherein the crystal of nitrobenzenesulfonamide derivative (2) is the crystal of the above-mentioned 1.
5. The production method of the above-mentioned 2, wherein the reaction of the (2R,3S)-3-benzyloxycarbonylamino-2-hydroxy-1-(N-isobutylamino)-4-phenylbutane with p-nitrobenzenesulfonyl chloride is carried out in ethyl acetate, isopropyl acetate or a mixed solvent thereof.
6. The production method of the above-mentioned 2, wherein the organic layer is cooled to a temperature lower than 20°C in step (iii).
7. A production method of (2R,3S)-N-(3-amino-2-hydroxy-4-phenylbutyl)-N-isobutyl-4-aminobenzenesulfonamide represented by the formula (1) 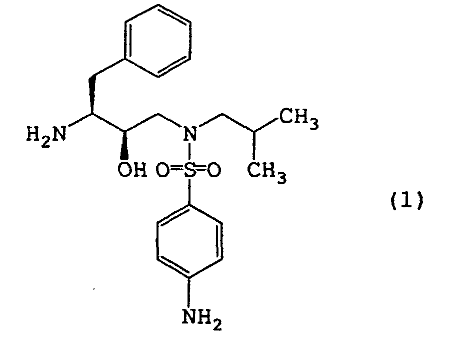 (benzenesulfonamide derivative (1)), which comprises reacting a crystal of nitrobenzenesulfonamide derivative (2) of the above-mentioned 1 with a hydrogen in the presence of a palladium catalyst.
8. The production method of benzenesulfonamide derivative (1), which comprises obtaining a crystal of nitrobenzenesulfonamide derivative (2) according to any of the above-mentioned 2 to 7, and reacting the crystal with a hydrogen in the presence of a palladium catalyst.
9. The production method of the above-mentioned 7 or 8, which further comprises a step of crystallizing benzenesulfonamide derivative (1) using a polar solvent as a good solvent and water as a poor solvent.
10. The production method of the above-mentioned 7 or 8, wherein the reaction of the crystal of nitrobenzenesulfonamide derivative (2) with a hydrogen is carried out in a polar solvent.
11. The production method of the above-mentioned 9 or 10, wherein the polar solvent is alcohol or a mixed solvent of alcohol and water.
12. A production method of a crystal of benzenesulfonamide derivative (1), which comprises crystallizing benzenesulfonamide derivative (1) using a polar solvent as a good solvent and water as a poor solvent.
13. The production method of the above-mentioned 12, wherein the nitrobenzenesulfonamide derivative (2) is reacted with a hydrogen in the presence of a palladium catalyst to give benzenesulfonamide derivative (1), which is then crystallized using a polar solvent as a good solvent and water as a poor solvent.
14. The production method of the above-mentioned 13, wherein the nitrobenzenesulfonamide derivative (2) is reacted with a hydrogen in the presence of a palladium catalyst and an acid to give a salt of benzenesulfonamide derivative (1), which is neutralized with alkali and then crystallized using a polar solvent as a good solvent and water as a poor solvent.
15. The production method of the above-mentioned 13 or 14, wherein the reaction of nitrobenzenesulfonamide derivative (2) with a hydrogen is carried out in a polar solvent.
16. The production method of the above-mentioned 12 to 15, wherein the polar solvent is alcohol or a mixed solvent of alcohol and water.

### Brief Description of the Drawings

Fig. 1 shows a powder X-ray diffraction analysis chart of nitrobenzenesulfonamide derivative (2) obtained in Example 6, wherein the vertical axis shows intensity and the transverse axis shows a diffraction angle (2θ).
Fig. 2 shows a powder X-ray diffraction analysis chart of nitrobenzenesulfonamide derivative (2) obtained in Comparative Example 1, wherein the vertical axis shows intensity and the transverse axis shows a diffraction angle (2θ).

### Embodiment of the Invention

The present invention is explained in detail in the following.

### nitrobenzenesulfonamide derivative (2)

(2R,3S)-N-(3-Benzyloxycarbonylamino-2-hydroxy-4-phenylbutyl)-N-isobutyl-4-nitrobenzenesulfonamide represented by the following formula (2) 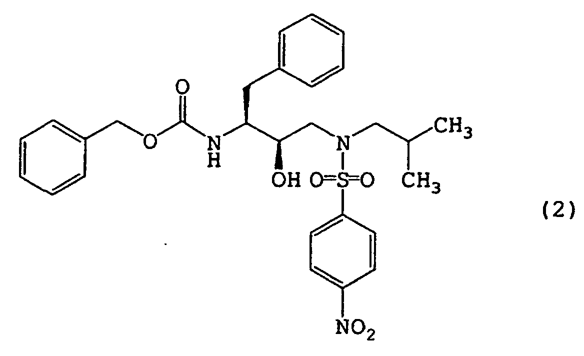 is a known compound, and is known to be produced by, for example, reacting compound (3) with p-nitrobenzenesulfonyl chloride, as shown in the following scheme.

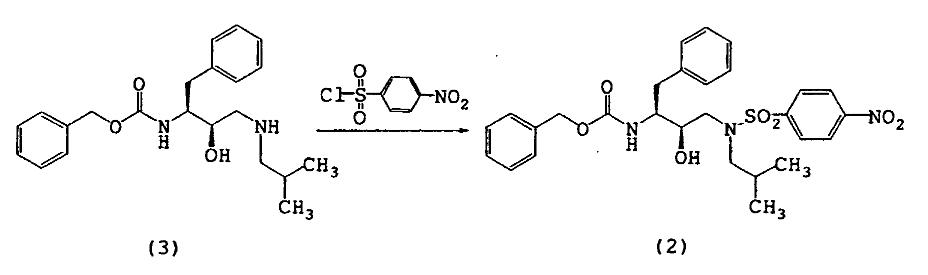

The production of nitrobenzenesulfonamide derivative (2) can be performed, for example, according to the methods described in the aforementioned reference and JP-A-9-194444.

The production of nitrobenzenesulfonamide derivative (2) is generally performed in the presence of a base. As the base, for example, triethylamine, diisopropylethylamine, pyrimidine, 2,6-lutidine, 4-(dimethylamino)pyridine (DMAP) and the like can be mentioned, with preference given to triethylamine. The amount of the base to be used is generally 1-5 equivalents, preferably 1-3 equivalents, relative to compound (3).

The amount of p-nitrobenzenesulfonyl chloride to be used for the production of nitrobenzenesulfonamide derivative (2) is generally 1-2 equivalents, preferably 1-1.5 equivalents, relative to compound (3).

The reaction solvent for nitrobenzenesulfonamide derivative (2) is, for example, ethyl acetate, isopropyl acetate, dichloromethane, dichloroethane, toluene and the like, which may be used alone or as a mixed solvent. Because a reaction solvent does not need to be removed when the isolation and purification step to be mentioned below is applied, ethyl acetate and isopropyl acetate are preferable. The total amount of the solvent to be used is generally 3-15 ml, preferably 5-10 ml, relative to 1 g of compound (3).

The reaction of compound (3) with p-nitrobenzenesulfonyl chloride is carried out generally at 10-80°C, preferably 30-60°C, generally for 1-10 hr, preferably 1-5 hr.

In addition, compound (3) can be also produced by reacting, for example, (2S,3S)-3-benzyloxycarbonylamino-1,2-epoxy-4-phenylbutane with isobutylamine (e.g., JP-A-9-194444, Examples 20, 21).

The present inventors have studied the production methods of benzenesulfonamide derivative (1) on an industrial scale, and found that, by using a particular crystal of nitrobenzenesulfonamide derivative (2) as a starting material of the catalytic reduction step, the reduction reaction proceeds rapidly, and the conventional problems in the catalytic reduction reaction (catalytic reduction reaction (hydrogenation reaction) proceeds markedly poorly, thereby causing an increased amount of catalytic reduction catalysts to be added, a longer reaction time, a lower yield and the like) can be solved. The detail is as follows.
(A) The present inventors have found that, while not known before, nitrobenzenesulfonamide derivative (2) has crystal polymorphism, and by using, out of crystal forms, crystal A specified below as a starting material of a catalytic reduction reaction, the above-mentioned conventional problems in the catalytic reduction reaction can be solved. Accordingly, crystal A is particularly useful as an intermediate for benzenesulfonamide derivative (1). When measured by the powder X-ray diffraction analysis method (2θ, CuKα radiation), crystal A shows strong peaks at 6.8°, 14.2° and 20.9°, moderate peaks at 8.1°, 13.6°, 16.1°, 18.6° and 20.4°, and weak peaks at 11.0°, 19.1°, 22.1°, 24.2°, 25.4°, 25.7°, 27.6° and 28.6°. In this way, crystal A is characterized by strong diffraction X-ray peaks (6.8°, 14.2° and 20.9°).
(B) As a production method of crystal A, for example, a method comprising subjecting a reaction solution to dehydration drying and then adding heptane or hexane, which is a poor solvent to a benzenesulfonamide derivative, to allow for crystallization can be mentioned. According to the finding of the present inventors, when crystallization is conducted at a temperature exceeding 20°C, the possibility of producing crystal A increases as the temperature becomes higher. When the crystallization is conducted at a temperature exceeding 30°C, crystal A tends to be obtained even without dehydration drying of the reaction solution. From the aspects of crystallization yield, the amount of a poor solvent to be used and the like, a method comprising dehydration drying of the reaction solution, followed by cooling crystallization, is preferable, and this method is highly advantageous for the production on an industrial scale.
(C) At a laboratory level, a drying agent such as sodium sulfate, magnesium sulfate and the like is often used for dehydration drying of an organic solvent. However, employing such method for an industrial scale production is practically difficult in view of the cost. The present inventors have studied a dehydration drying method applicable even on an industrial scale and, as a result, found that the following method can efficiently remove water from a reaction solution, based on which obtained crystal A stably by cooling crystallization as well.
   A production method of a crystal of (2R,3S)-N-(3-benzyloxycarbonylamino-2-hydroxy-4-phenylbutyl)-N-isobutyl-4-nitrobenzenesulfonamide, which comprises subjecting a reaction mixture, obtained by reacting (2R,3S)-3-benzyloxycarbonylamino-2-hydroxy-1-(N-isobutylamino)-4-phenylbutane with p-nitrobenzenesulfonyl chloride, successively to step (i), step (ii) and step (iii), or successively to step (i), step (ii') and step (iii):
   (i) a step of adding water to carry out partitioning,
   (ii) a step of adding either or both of heptane and hexane to an organic layer obtained in the previous step to carry out partitioning,
   (ii') a step of washing the organic layer obtained in the previous step with brine, and adding either or both of heptane and hexane thereto, and
   (iii) a step of cooling the organic layer obtained in the previous step.
(D) In the method of the above-mentioned (C), as a solvent that can be preferably used for both a reaction solvent used for the reaction of compound (3) with p-nitrobenzenesulfonyl chloride (same as 4-nitrobenzenesulfonyl chloride) and as a crystallization solvent used for the crystallization of nitrobenzenesulfonamide derivative (2), ethyl acetate and isopropyl acetate can be mentioned. If a reaction solvent can be used as a crystallization solvent, a method suitable for an industrial scale production can be achieved, since evaporation and substitution of solvent after reaction is not necessary and crystallization can be continuously performed after the reaction. While the reaction solution can be concentrated and subjected to a crystallization step to increase the crystallization yield, since the crystallization yield can be also controlled through cooling temperature or the amount of poor solvent to be added, such step is not particularly necessary in the present invention.

Steps (i)-(iii) and (ii') are explained in detail in the following.

### Step (i)

Step (i) is necessary for removing a salt byproduced during the reaction of compound (3) with p-nitrobenzenesulfonyl chloride in the presence of a base. The water contained in the organic layer, which is the main cause of producing crystal B, which is inconvenient for the purification of nitrobenzenesulfonamide derivative (2), mostly gets mixed in during this step.

The amount of water to be used in Step (i) is generally 3-15 ml, preferably 3-10 ml, relative to 1 g of compound (3).

Step (i) completes by partitioning after mixing at generally 10-80°C, preferably 30-60°C.

### Step (ii)

In Step (ii), either or both of heptane and hexane is/are used. From the aspect of operation environments, however, heptane is most preferably used. The amount of heptane and hexane to be added is not particularly limited as long as it does not cause precipitation of nitrobenzenesulfonamide derivative (2), but the amount is generally 1-10 ml, preferably 2-10 ml, relative to 1 g of compound (3). When both heptane and hexane are used, the total amount needs only to be within the above-mentioned range. When the amount of use exceeds this range, crystals tend to be precipitated, and when the crystals are precipitated, the crystals may possibly be crystal B. When the amount is lower than this range, partitioning becomes difficult, and the object product is finally obtained as crystal B.

Step (ii) includes adding either or both of heptane and hexane to the organic layer obtained in the aforementioned step, stirring the mixture generally at 10-90°C, preferably 30-70°C, and is completed by partitioning the mixture.

After subjecting to Step (ii), either or both of heptane and hexane is/are further added to the obtained organic layer to lower the solubility of the object product, which is then subjected to Step (iii), whereby the crystallization yield of the object product can be increased further. The amount of heptane and hexane to be added here is an amount that makes the amount of hexane and heptane in the organic layer to be subjected to Step (iii) generally 2-15 ml, preferably 3-10 ml, relative to 1 g of compound (3). When the organic layer to be subjected to Step (iii) contains both heptane and hexane, the total amount thereof needs only to be within the above-mentioned range. When the amount of heptane and hexane is lower than the above-mentioned range, the crystallization yield becomes low, and when the amount exceeds this range, the crystallization yield does not increase, which is not economical.

### Step (ii')

Step (ii') is generally conducted at 10-80°C, preferably 30-60°C.

The brine to be used in Step (ii') has a concentration of generally not less than 15 wt% when added to the organic layer, and is saturated or below saturation, and particularly preferably saturated. The amount of use thereof when saturated brine is used is, for example, generally 1-15 ml, preferably 1-10 ml, relative to 1 g of compound (3).

The amount of heptane and hexane to be added in Step (ii') is generally 2-15 ml, preferably 3-10 ml, relative to 1 g of compound (3). When both heptane and hexane are added, the total amount needs only to be within the above-mentioned range. When the amount of heptane and hexane is lower than the above-mentioned range, the crystallization yield becomes low, and when the amount exceeds this range, the crystallization yield does not increase, which is not economical.

In Step (ii'), when water remains in the organic layer after washing with brine, the remaining water can be also removed by partitioning after adding hexane and heptane.

When the organic layer obtained in Step (i) is subjected to Step (iii) as it is without subjecting to Step (ii) and Step (ii'), the object product tends to be obtained as crystal B. Particularly, when the crystallization is performed at a temperature lower than 30°C, particularly lower than 20°C, such tendency becomes noticeable. When the cooling crystallization is performed at not lower than 20°C and lower than 30°C, the possibility of producing a mixed crystal of crystal A and crystal B becomes high, though subject to change depending on the water content of the organic solvent to be subjected to crystallization. In this temperature range, moreover, crystal A tends to shift to crystal B. As compared to crystal A, crystal B shows markedly inferior filterability and lower purification efficiency, which in turn causes residual p-nitrobenzenesulfonyl chloride in the crystal. As a result, the reduction reaction is inhibited and does not proceed smoothly, which gives rise to problems such as a longer reaction time, an increased amount of catalytic reduction catalysts to be added and the like.

However, by subjecting to step (iii) after removing water in the organic layer obtained in step (i) by step (ii) or step (ii'), the object product is obtained as crystal A regardless of the crystallization temperature, which affords highly efficient progress of the reduction reaction.

As shown above, Step (ii) and Step (ii') are very important and indispensable steps in the present invention. Of the Step (ii) and Step (ii'), Step (ii) is preferable in view of the convenience of operation and the like.

### Step (iii)

While the cooling temperature in Step (iii) depends on the composition and amount of the solvent and the like, it needs only to be a temperature at which the object product is efficiently crystallized, and is preferably not higher than 30°C, more preferably -10°C to 30°C.

After the completion of Step (iii), nitrobenzenesulfonamide derivative (2) can be purified by conventional methods such as filtration and the like. Where necessary, the purity can be increased further by subjecting the obtained crystal to conventional methods such as washing and the like.

The majority of p-nitrobenzenesulfonyl chloride contained in a reaction solution is converted to p-nitrobenzenesulfonic acid by Step (i). Since p-nitrobenzenesulfonic acid becomes a factor that decreases purity of nitrobenzenesulfonamide derivative (2) in the subsequent Steps (ii), (ii') and (iii), it is preferably removed after the completion of Step (i). While the method for removal is not particularly limited, a method comprising washing the organic layer obtained in Step (i) with an aqueous sodium hydrogen carbonate solution is also industrially suitable and preferable in view of convenience and economical aspect. The concentration of an aqueous sodium hydrogen carbonate solution when added to the organic layer is not particularly limited, and preferably not less than 5 wt%, and is saturated or below saturation, and particularly preferably saturated.

Washing is generally conducted at 10°C to 80°C, preferably 30°C to 60°C.

The aqueous sodium hydrogen carbonate solution to be used for washing is preferably adjusted in advance to a washing temperature.

### Benzenesulfonamide derivative (1)

The benzenesulfonamide derivative (1) can be produced by subjecting nitrobenzenesulfonamide derivative (2) to hydrogenation using a palladium catalyst, and simultaneously performing elimination (deprotection) of amino protecting group (benzyloxycarbonyl) and reduction of nitro group (WO96/28418 (US Patent No. 6,140,505), Example 21).

While benzenesulfonamide derivative (1) can be also produced using a solvent other than a polar solvent, a catalytic reduction using a polar solvent, which is a preferable embodiment, is explained here.

To be specific, nitrobenzenesulfonamide derivative (2) is dissolved or suspended in a polar solvent. While the temperature of suspending or dissolving is not particularly limited, it is preferably 0°C to 100°C, more preferably 20°C to 80°C.

As the polar solvent in the present invention, a single or mixed organic solvent (except water alone) miscible with water can be mentioned, such as alcohols (e.g., methanol, ethanol, 1-propanol, 2-propanol etc.) and any mixed organic solvent of these solvents. As long as the effect of the present invention is not inhibited, water may be present in the above-mentioned organic solvent, and a mixed solvent with water can be included in the polar solvent as used herein. As the polar solvent, methanol and ethanol are preferable, and methanol is particularly preferable. While the amount of the polar solvent to be used is not particularly limited, generally 3-50 ml, preferably 5-15 ml, of a solvent relative to 1 g of nitrobenzenesulfonamide derivative (2) can be used.

Then, the solution or suspension is subjected to catalytic reduction. To be specific, a palladium catalyst is added to the solution or suspension and hydrogen pressure is applied. While the hydrogen pressure during catalytic reduction is not particularly limited as long as the reaction proceeds smoothly, it is preferably 1-30 atm, more preferably 1-10 atm. While the reaction temperature is not particularly limited as long as the reaction proceeds and the resulting product is not decomposed, it is generally 0°C-80°C, preferably 20°C-60°C. The reaction time is generally 2-48 hr, preferably 3-24 hr.

As the palladium catalyst, palladium on carbon and palladium hydroxide on carbon can be preferably mentioned. The amount of the palladium catalyst to be used is generally 0.1-20 mol, preferably 0.5-10 mol, relative to 100 mol of nitrobenzenesulfonamide derivative (2).

While not necessary when crystal A is used as nitrobenzenesulfonamide derivative (2), when crystal A is not used (e.g., a reaction solution obtained without isolation and purification of nitrobenzenesulfonamide derivative (2) after reaction of compound (3) with p-nitrobenzenesulfonyl chloride, crystal B, mixed crystals of crystal B and crystal A and the like), the reaction may be carried out in the presence of an acid to accelerate catalytic reduction reaction (reduction of nitro group and elimination of benzyloxycarbonyl group). As the acid, for example, inorganic acids such as hydrochloric acid, sulfuric acid, hydrobromic acid and the like, organic acids such as acetic acid, methanesulfonic acid and the like can be mentioned, which is particularly preferably hydrochloric acid. The acid is used in an amount of generally 1-5 equivalents, preferably 1-3 equivalents, relative to nitrobenzenesulfonamide derivative (2).

When an acid is made to be present in the reaction system, benzenesulfonamide derivative (1) is present as a salt after the completion of the reaction, and to obtain benzenesulfonamide derivative (1) as a free amine, the salt needs to be neutralized. As the base to be used for neutralization, for example, inorganic bases such as sodium hydroxide, potassium hydroxide, sodium carbonate, potassium carbonate, sodium hydrogencarbonate, potassium hydrogen carbonate and the like can be mentioned, which is particularly preferably sodium hydroxide. The base is not particularly limited as long as it is used in an amount that permits neutralization of the salt.

It is also preferable to add water during reduction of nitrobenzenesulfonamide derivative (2), because it shortens the reaction time. Water is generally added before adding a palladium catalyst. The amount of water to be used is not particularly limited as long as nitrobenzenesulfonamide derivative (2) is not precipitated, and water can be added in the amount of generally 0.1-5 ml, preferably 0.1-3 ml, relative to 1 g of nitrobenzenesulfonamide derivative (2).

While benzenesulfonamide derivative (1) can be isolated and purified by a conventional method after the completion of reduction, by subjecting the derivative to the following crystallization method of the present invention, highly pure benzenesulfonamide derivative (1) can be obtained efficiently.

In the following crystallization step of the present invention, a polar solvent is used as a good solvent. Thus, a polar solvent is also preferably used as a reaction solvent of the catalytic reduction step. By using a polar solvent as a reaction solvent of catalytic reduction step, after filtering off the palladium catalyst from the reaction solution, the filtrate can be subjected as it is to a crystallization step, which can more efficiently afford benzenesulfonamide derivative (1) as a crystal. It is also possible to wash the palladium catalyst removed by filtration as necessary with an organic solvent (preferably a polar solvent) and include the washing in a solution to be subjected to a crystallization step. Needless to say, it is possible to concentrate the filtrate after removal of the palladium catalyst and then subject the filtrate to the following novel crystallization method, but this is not preferable in view of efficiency and economical aspect.

A novel crystallization method capable of efficiently removing impurity from low purity benzenesulfonamide derivative (1) to improve purity is explained in the following.

A crystallization step of benzenesulfonamide derivative (1) using a polar solvent as a good solvent and water as a poor solvent is explained.

As a crystallization step of benzenesulfonamide derivative (1) using a polar solvent as a good solvent and water as a poor solvent, for example,
(1) a method comprising adding water to a solution of a polar solvent wherein benzenesulfonamide derivative (1) has been dissolved (where necessary, cooling crystallization, concentration crystallization etc. may be combined),
(2) a method comprising subjecting a solution of a mixed solvent of water and a polar solvent, in which benzenesulfonamide derivative (1) has been dissolved, to cooling crystallization, concentration crystallization and the like (where necessary, a step of adding water and the like may be combined) ,
and the like can be mentioned.

As preferable examples of the polar solvent to be used for crystallization, methanol, ethanol, 2-propanol and the like can be mentioned. The amount of the polar solvent to be used is generally 3-20 ml, preferably 3-15 ml, relative to 1 g of benzenesulfonamide derivative (1).

To be precise, method (1) comprises dissolving the object product in a polar solvent and then adding water to this solution to allow for crystallization of the object product. To improve the yield, the mixture is heated when the object product is dissolved in a polar solvent.

In method (1), while the amount of water to be added is not particularly limited, it is preferably 10-200%, more preferably 20-100%, in a volume ratio relative to a polar solvent.

In method (1), crystallization, namely, addition of water, is preferably conducted at -10°C to 80°C, particularly preferably from 0°C to 70°C. While the temperature of water to be added is not particularly limited, it is preferably set in advance to a temperature at which to perform crystallization. In addition, while the method of adding water is not particularly limited, water can be added over a time period that does not cause temperature fall, and preferably added gradually generally over 30 min to 6 hr.

In method (1), addition of seed crystals after addition of water can further improve the yield.

The method (2) comprises dissolving the object product in a mixed solvent of water and a polar solvent, and subjecting this solution to cooling crystallization, concentration crystallization and the like to allow for crystallization of the object product. To improve the yield, water may be further added as necessary after dissolution of the object product in a mixed solvent of water and a polar solvent.

As the mixed solvent of water and a polar solvent in method (2), a solvent containing water in a volume ratio of preferably 10-200%, more preferably 20-150%, relative to the polar solvent can be used.

The cooling temperature when subjecting the solution to cooling crystallization depends on the solvent to be used and needs only to be such temperature as allows crystallization of the object product. In the case of a mixed solvent having a ratio within the above-mentioned range, the cooling temperature is generally -10 to 100°C, preferably 0 to 80°C.

To enhance the purification effect of method (2), it is preferable to heat the solution (about 30°C - 80°C) (the crystal is preferably dissolved completely when the crystal is present in the polar solvent solution), add water as necessary (in this case, it is preferable to avoid precipitation of crystal), and then cool the solution (-10°C to 25°C) to perform crystallization (cooling crystallization).

The crystallization may be performed with stirring or under standing still conditions. Where necessary, by adding seed crystals during addition or after addition of water, crystallization can be performed more easily. Where necessary, moreover, crystallization may be performed at a lower temperature by cooling during addition or after addition of water.

After crystallization by method (1) and (2), the obtained crystals are collected by filtration, whereby the object product alone can be extracted. This is because a polar impurity is dissolved in the mother liquor during crystallization. As a result, by the crystallization method of the present invention, highly pure benzenesulfonamide derivative (1) can be efficiently obtained.

In addition, the obtained crystals can be washed with water or a mixed solvent of alcohol and water. As the alcohol to be used here, for example, methanol, ethanol, 1-propanol, 2-propanol and the like can be mentioned. In addition, crystals can be also washed with an organic solvent such as heptane, hexane and the like. These solvents to be used for washing are preferably cooled in advance.

In addition, by crystallizing the obtained crystals of benzenesulfonamide derivative (1) again, as described in the aforementioned method (2) from a mixed solvent of water and a polar solvent (e.g., water:polar solvent (volume ratio) preferably 5:1-1:3), crystals having a higher purity can be obtained.

The benzenesulfonamide derivative (1) can be led to a pharmaceutical compound such as an anti-HIV protease inhibitor and the like according to the method described in Bioorganic & Medicinal Chemistry Letters, 1998, 8, pp. 687-689 and the like.

### Examples

The present invention is explained in more detail by referring to Examples. These Examples are not to be construed as limiting the present invention. In the following, wt% means % by weight.

Note that the powder X-ray diffraction analysis in Examples and Comparative Examples was performed using CuKα radiation.

### <Reference Example 1>

### Production of (2R,3S)-N-(3-benzyloxycarbonylamino-2-hydroxy-4-phenylbutyl)-N-isobutyl-4-nitrobenzenesulfonamide (nitrobenzenesulfonamide derivative (2))

Ethyl acetate (150 ml) was added to (2R,3S)-3-benzyloxycarbonylamino-2-hydroxy-1-(N-isobutylamino)-4-phenylbutane (30.0 g) and the mixture was heated to 40°C. Triethylamine (13.5 ml) and p-nitrobenzenesulfonyl chloride (19.74 g) were added, and the mixture was stirred for 3 hr, after which water (150 ml) was added to carry out partitioning. The organic layer was washed with water (150 ml) and concentrated. The obtained solid was dried under reduced pressure to give 42.65 g of (2R,35)-N-(3-benzyloxycarbonylamino-2-hydroxy-4-phenylbutyl)-N-isobutyl-4-nitrobenzenesulfonamide as a pale-yellow crystal (yield 94.8%). Analysis by HPLC revealed HPLC purity of 90.3% (HPLC area ratio).
¹H-NMR(CDCl₃)δppm: 0.84(d,*J*=6.1Hz,3H), 0.86(d,*J*=6.3Hz,3H), 1.75-1.95(m,1H), 2.88(dd,*J*=7.5,14.1Hz,2H), 2.96(d,*J*=6.8Hz,2H), 3.00(dd,*J*=4.7,14.1Hz,1H), 2.90(bs,1H), 3.12-3.26(m,*2*H), 3.80-3.91(m,2H), 4.99(bd,*J*=8.7Hz,1H), 5.01(s,2H), 7.21-7.32(m,10H), 7.92(d,*J*=8.7Hz,2H), 8.29(d,*J*=8.7Hz,2H);
¹³C-NMR(CDCl₃)δppm: 20.2, 20.4, 27.4, 35.9, 53.2, 55.9, 58.2, 67.4, 72.6, 124.8, 127.2, 128.3, 128.7, 128.9, 129.0, 129.1, 129.8, 136.6, 137.6, 145.0, 150.4, 157.0.

### <Example 1>

### Production of (2R,3S)-N-(3-amino-2-hydroxy-4-phenylbutyl)-N-isobutyl-4-aminobenzenesulfonamide (benzenesulfonamide derivative (1))

Methanol (142 ml) was added to (2R,3S)-N-(3-benzyloxycarbonylamino-2-hydroxy-4-phenylbutyl)-N-isobutyl-4-nitrobenzenesulfonamide (14.2 g) produced in Reference Example 1 and suspended. Concentrated hydrochloric acid (6.8 ml) was added and, after forming an argon atmosphere, 20% palladium hydroxide on carbon (918 mg) was added. After forming a hydrogen atmosphere (hydrogen pressure: 1 atm), the mixture was stirred at 40°C for 21 hr. The catalyst was filtered off and washed with methanol (28 ml). A 6N aqueous sodium hydroxide solution (11 ml) was added to the reaction mixture and water (114 ml) was added at 4°C over 5.7 hr. The crystals were collected by filtration and washed with heptane (71 ml). The obtained crystals were dried under reduced pressure to give 7.1 g of (2R,3S)-N-(3-amino-2-hydroxy-4-phenylbutyl)-N-isobutyl-4-aminobenzenesulfonamide (yield 71%). Analysis by HPLC revealed HPLC purity of 98.7% (HPLC area ratio).
¹H-NMR(CDCl₃)δppm: 0.88(d,*J*=6.6Hz,3H), 0.93 (d,*J*=6.6Hz,3H) , 1.06 (bs,2H) , 1.87(m,1H), 2.48(dd,*J*=13.4,9.9Hz,1H), 2.82(dd,*J*=13.4,6.7Hz,1H), 2.96(dd,*J*=13.4,3.8Hz,1H), 3.01(dd,*J*=13.4,8.3Hz,1H), 3.11(m,1H), 3.16(dd,*J*=15.1,2.5Hz,1H), 3.28(dd,*J*=15.1,9.1Hz,1H), 3.56(bs,1H), 3.74(bs,1H), 4.17(bs,2H), 6.68(d,*J*=8.8Hz,2H), 7.20-7.24(m,3H), 7.29-7.33(m,2H), 7.58(d,*J*=8.8Hz,2H);
mass spectrum m/e: 392.21(MH⁺)

### <Example 2>

### Purification of (2R,3S)-N-(3-amino-2-hydroxy-4-phenylbutyl)-N-isobutyl-4-aminobenzenesulfonamide (benzenesulfonamide derivative (1))

2-Propanol (13.5 ml) and water (15 ml) were added and (2R,3S)-N-(3-amino-2-hydroxy-4-phenylbutyl)-N-isobutyl-4-aminobenzenesulfonamide (1.92 g) produced in Example 1 was dissolved at 50°C. The mixture was cooled to 10°C over 3 hr and the crystals were collected by filtration. The obtained crystals were washed with a 1:1 mixed solvent (8 ml) of cooled water and 2-propanol and dried under reduced pressure to give 1.58 g of (2R,3S)-N-(3-amino-2-hydroxy-4-phenylbutyl)-N-isobutyl-4-aminobenzenesulfonamide (yield 82%). Analysis by HPLC revealed HPLC purity of 99.9% (HPLC area ratio).

### <Reference Example 2>

### Production of (2R,3S)-N-(3-benzyloxycarbonylamino-2-hydroxy-4-phenylbutyl)-N-isobutyl-4-nitrobenzenesulfonamide (nitrobenzenesulfonamide derivative (2))

Ethyl acetate (150 ml) and triethylamine (13.5 ml) were added to (2R,3S)-3-benzyloxycarbonylamino-2-hydroxy-1-(N-isobutylamino)-4-phenylbutane (30.0 g) and the mixture was heated to 40°C. p-Nitrobenzenesulfonyl chloride (19.74 g) was added, and after stirring for 1.5 hr, water (150 ml) was added to carry out partitioning. The organic layer was washed with water (150 ml) and saturated aqueous sodium hydrogen carbonate solution (150 ml), after which ethyl acetate (30 ml) was added and the mixture was heated to 60°C. To this ethyl acetate solution was gradually added heptane (150 ml), crystals of the title compound were added and the mixture was cooled from 60°C to 0°C over 6 hr. After further stirring overnight, crystals were collected by filtration. The obtained crystals were washed with a 3:1 mixed solvent (90 ml) of heptane and ethyl acetate, and dried under reduced pressure to give 35.2 g of (2R,3S)-N-(3-benzyloxycarbonylamino-2-hydroxy-4-phenylbutyl)-N-isobutyl-4-nitrobenzenesulfonamide as white crystals (yield 78%). Analysis by HPLC revealed HPLC purity of 99.4% (HPLC area ratio).

### <Example 3>

### Purification of (2R,3S)-N-(3-amino-2-hydroxy-4-phenylbutyl)-N-isobutyl-4-aminobenzenesulfonamide (benzenesulfonamide derivative (1))

Methanol (150 ml) was added to (2R,3S)-N-(3-benzyloxycarbonylamino-2-hydroxy-4-phenylbutyl)-N-isobutyl-4-nitrobenzenesulfonamide (15.0 g) produced in Reference Example 2 and suspended at room temperature. After forming an argon atmosphere, 20% palladium hydroxide on carbon (379 mg) was added, and after forming a hydrogen atmosphere (hydrogen pressure 1 atm), the mixture was stirred at 40°C for 3.5 hr. The catalyst was filtered off and washed with methanol (30 ml). The reaction mixture was heated to 60°C and water (150 ml) was gradually added (over a time that does not decrease the temperature). Crystals of the title compound were added, and the mixture was cooled from 60°C to 0°C over 6 hr and stirred further'overnight. The crystals were collected by filtration from this slurry and the obtained crystals were washed with a 2:1 mixed solvent (30 ml) of water and methanol and dried under reduced pressure to give 9.57 g of (2R,3S)-N-(3-amino-2-hydroxy-4-phenylbutyl)-N-isobutyl-4-aminobenzenesulfonamide as white crystals (yield 91%). Analysis by HPLC revealed HPLC purity of 99.95% (HPLC area ratio).

### <Example 4>

### Purification of (2R,3S)-N-(3-amino-2-hydroxy-4-phenylbutyl)-N-isobutyl-4-aminobenzenesulfonamide (benzenesulfonamide derivative (1))

Methanol (75 ml) was added to (2R,3S)-N-(3-benzyloxycarbonylamino-2-hydroxy-4-phenylbutyl)-N-isobutyl-4-nitrobenzenesulfonamide (15.0 g) produced in Reference Example 2 and suspended at room temperature. After forming an argon atmosphere, 20% palladium hydroxide on carbon (379 mg) was added, and after forming a hydrogen atmosphere (hydrogen pressure 1 atm), the mixture was stirred at 40°C for 5.5 hr. The catalyst was filtered off and washed with methanol (15 ml). The reaction mixture was heated to 70°C and water (58.5 ml) was gradually added (over a time that does not decrease the temperature). Crystals of the title compound were added and the mixture was cooled from 70°C to 0°C over 7 hr and stirred further overnight. The crystals were collected by filtration from this slurry, washed with a 2:1 mixed solvent (30 ml) of water and methanol and dried under reduced pressure to give 9.77 g of (2R,3S)-N-(3-amino-2-hydroxy-4-phenylbutyl)-N-isobutyl-4-aminobenzenesulfonamide as white crystals (yield 92%). Analysis by HPLC revealed HPLC purity of 99.95% (HPLC area ratio).

### <Example 5>

### Production of (2R,3S)-N-(3-amino-2-hydroxy-4-phenylbutyl)-N-isobutyl-4-aminobenzenesulfonamide (benzenesulfonamide derivative (1))

Methanol (25 ml) and water (2.5 ml) were added to (2R,3S)-N-(3-benzyloxycarbonylamino-2-hydroxy-4-phenylbutyl)-N-isobutyl-4-nitrobenzenesulfonamide (5.0 g) and after forming an argon atmosphere, 20% palladium hydroxide on carbon (water 31%, 91.4 mg) was added. After forming a hydrogen atmosphere (hydrogen pressure 1 atm), the mixture was stirred at 40°C for 3 hr. The catalyst was filtered off and washed with methanol (5 ml). The reaction mixture was heated to 70°C and water (17 ml) was gradually added (over a time that does not decrease the temperature), cooled to 0°C and crystals were collected by filtration. The obtained crystals were washed with a mixed solvent of water (3.3 ml) and methanol (6.6 ml). After further washing with water (25 ml), the crystals were dried under reduced pressure to give 3.16 g of (2R,3S)-N-(3-amino-2-hydroxy-4-phenylbutyl)-N-isobutyl-4-aminobenzenesulfonamide as white crystals (yield 90%).

The properties of the resulting product were the same as in Example 1.

### <Example 6>

### Production of (2R,3S)-N-(3-benzyloxycarbonylamino-2-hydroxy-4-phenylbutyl)-N-isobutyl-4-nitrobenzenesulfonamide (nitrobenzenesulfonamide derivative (2))

Ethyl acetate (48 ml) and triethylamine (3.6 ml) were added to (2R,3S)-3-benzyloxycarbonylamino-2-hydroxy-1-(N-isobutylamino)-4-phenylbutane (8.0 g) and the mixture was heated to 40°C. p-Nitrobenzenesulfonyl chloride (5.27 g) was added and the mixture was stirred for 2 hr, after which water (40 ml) was added to carry out partitioning. The organic layer was washed with saturated aqueous sodium hydrogen carbonate solution (40 ml) and saturated brine (10 ml) and heated to 60°C, and heptane (40 ml) was added. The mixture was cooled to 0°C and crystals were collected by filtration. The obtained crystals were washed with a mixed solvent of heptane (18 ml) and ethyl acetate (6 ml), and dried under reduced pressure to give 9.17 g of (2R,3S)-N-(3-benzyloxycarbonylamino-2-hydroxy-4-phenylbutyl)-N-isobutyl-4-nitrobenzenesulfonamide as crystals (yield 76%, crystal A).

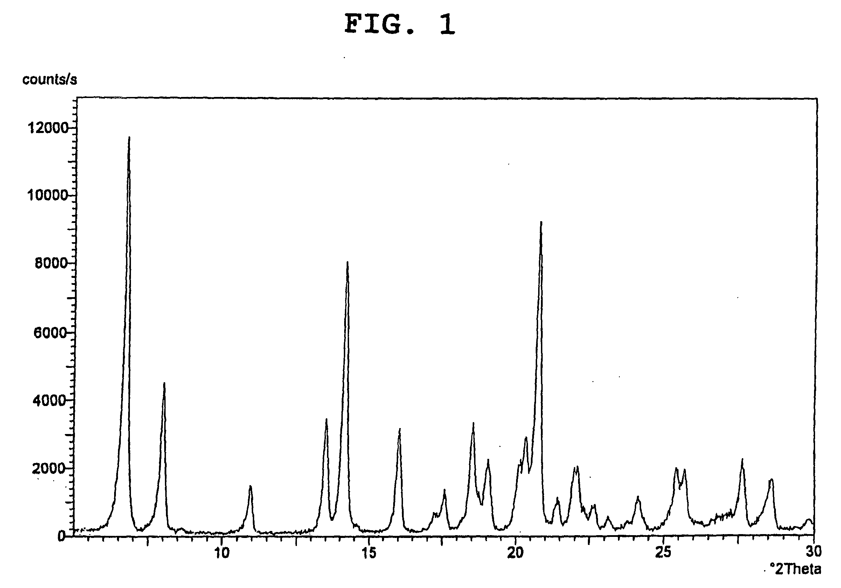

The property data of the obtained crystals were the same as in Reference Example 1. The measurement results of powder X-ray diffraction analysis of CuKα radiation are shown in Fig. 1. The major diffraction angles 2θ(°) are as follows;
6.8, 8.1, 11.0, 13.6, 14.2, 16.1, 18.6, 19.1, 20.4, 20.9, 22.1, 24.2, 25.4, 25.7, 27.6, 28.6.

### <Example 7>

### Production of (2R,3S)-N-(3-benzyloxycarbonylamino-2-hydroxy-4-phenylbutyl)-N-isobutyl-4-nitrobenzenesulfonamide (nitrobenzenesulfonamide derivative (2))

Ethyl acetate (24 ml) and triethylamine (1.8 ml) were added to (2R,3S)-3-benzyloxycarbonylamino-2-hydroxy-1-(N-isobutylamino)-4-phenylbutane (4.0 g) and the mixture was heated to 40°C. p-Nitrobenzenesulfonyl chloride (2.64 g) was added and the mixture was stirred for 2 hr, after which water (20 ml) was added carry out partitioning. The organic layer was washed with saturated aqueous sodium hydrogen carbonate solution (20 ml) and heated to 60°C. Heptane (8 ml) was added and the aqueous layer was separated. Heptane (12 ml) was further added and the mixture was cooled to 0°C. Crystals were collected by filtration, washed with a mixed solvent of heptane (9 ml) and ethyl acetate (3 ml), and dried under reduced pressure to give 4.8 g of (2R,3S)-N-(3-benzyloxycarbonylamino-2-hydroxy-4-phenylbutyl)-N-isobutyl-4-nitrobenzenesulfonamide as crystals (yield 80%, crystal A). The major diffraction angles 2θ(°) measured by powder X-ray diffraction analysis of CuKα radiation were the same as in Example 6.

### <Example 8>

### Production of (2R,3S)-N-(3-benzyloxycarbonylamino-2-hydroxy-4-phenylbutyl)-N-isobutyl-4-nitrobenzenesulfonamide (nitrobenzenesulfonamide derivative (2))

Ethyl acetate (6 ml) was added to (2R,3S)-3-benzyloxycarbonylamino-2-hydroxy-1-(N-isobutylamino)-4-phenylbutane (1.0 g) and the mixture was heated to 40°C. Triethylamine (0.452 ml) and p-nitrobenzenesulfonyl chloride (0.658 g) were added and the mixture was stirred for 2 hr, after which water (5 ml) was added to carry out partitioning. The organic layer was washed with saturated aqueous sodium hydrogen carbonate solution (5 ml) and heated to 60°C. Heptane (5 ml) was added and the aqueous layer was separated. Heptane (5 ml) was further added and the mixture was cooled to 30°C. Crystals were collected by filtration, washed with a mixed solvent of heptane (3.75 ml) and ethyl acetate (1.25 ml), and dried under reduced pressure to give 1.07 g of (2R,3S)-N-(3-benzyloxycarbonylamino-2-hydroxy-4-phenylbutyl)-N-isobutyl-4-nitrobenzenesulfonamide as crystals (yield 71%, crystal A). The major diffraction angles 2θ(°) measured by powder X-ray diffraction analysis of CuKα radiation were the same as in Example 6.

### <Comparative Example 1>

### Production of (2R,3S)-N-(3-benzyloxycarbonylamino-2-hydroxy-4-phenylbutyl)-N-isobutyl-4-nitrobenzenesulfonamide (nitrobenzenesulfonamide derivative (2))

Ethyl acetate (18 ml) and triethylamine (1.36 ml) were added to (2R,3S)-3-benzyloxycarbonylamino-2-hydroxy-1-(N-isobutylamino)-4-phenylbutane (3.0 g) and the mixture was heated to 40°C. p-Nitrobenzenesulfonyl chloride (1.97 g) was added and the mixture was stirred for 1 hr 45 min, after which water (9 ml) was added to carry out partitioning. The organic layer was washed with saturated aqueous sodium hydrogen carbonate solution (9 ml) and heated to 50°C. Heptane (15 ml) was added and the mixture was cooled to 0°C. Crystals were collected by filtration, washed with a mixed solvent of heptane (11.3 ml) and ethyl acetate (3.8 ml), and dried under reduced pressure to give 3.30 g of (2R,3S)-N-(3-benzyloxycarbonylamino-2-hydroxy-4-phenylbutyl)-N-isobutyl-4-nitrobenzenesulfonamide as crystals (yield 73%, crystal B).

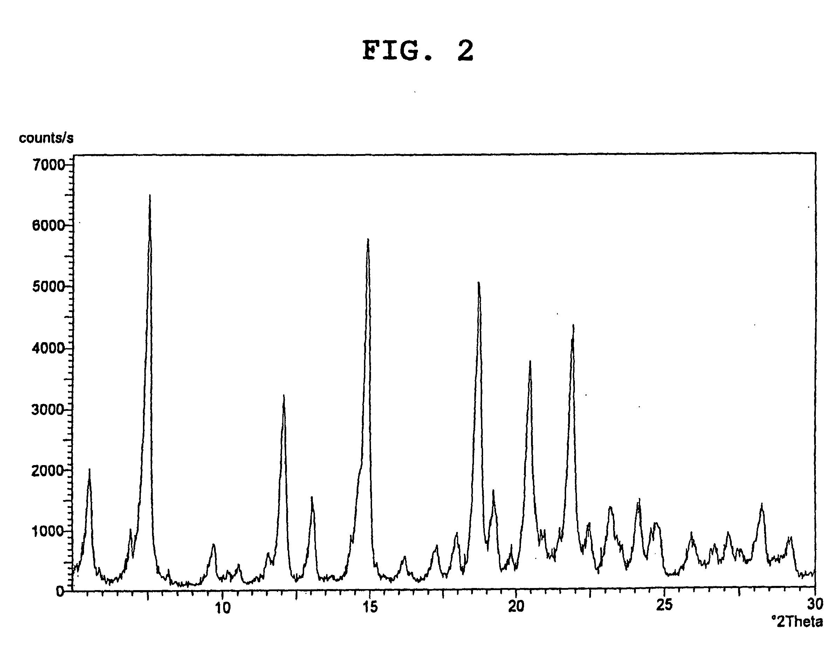

The property data of the obtained crystals were the same as in Reference Example 1. The measurement results of powder X-ray diffraction analysis of CuKα radiation are shown in Fig. 2. The major diffraction angles 2θ(°) are as follows;
5.6, 7.6, 12.1, 13.1, 14.9, 18.7, 19.2, 20.5, 21.9, 23.2, 24.1, 24.8, 28.2.

### <Example 9>

### Production of (2R,3S)-N-(3-amino-2-hydroxy-4-phenylbutyl)-N-isobutyl-4-aminobenzenesulfonamide (benzenesulfonamide derivative (1))

Ethyl acetate (18 ml) was added to (2R,3S)-3-benzyloxycarbonylamino-2-hydroxy-1-(N-isobutylamino)-4-phenylbutane (3.0 g) and the mixture was heated to 40°C. Triethylamine (1.36 ml) and p-nitrobenzenesulfonyl chloride (1.97 g) were added and the mixture was stirred for 3 hr, after which water (9 ml) was added to carry out partitioning. The organic layer was washed with saturated aqueous sodium hydrogen carbonate solution (9 ml) and heated to 60°C. Heptane (30 ml) was added and the aqueous layer was separated. The solution was cooled to 30°C and crystals were collected by filtration. The obtained crystals were washed with a mixed solvent of heptane (11.25 ml) and ethyl acetate (3.75 ml), and dried under reduced pressure to give 3.67 g of (2R,3S)-N-(3-benzyloxycarbonylamino-2-hydroxy-4-phenylbutyl)-N-isobutyl-4-nitrobenzenesulfonamide as crystals (yield 82%, crystal A). The property data of the obtained crystals were the same as in Example 6. Methanol (15 ml) and water (1.5 ml) were added to the obtained (2R,3S)-N-(3-benzyloxycarbonylamino-2-hydroxy-4-phenylbutyl)-N-isobutyl-4-nitrobenzenesulfonamide (3.0 g) and after forming an argon atmosphere, 5% palladium hydroxide on carbon (water 54.7 wt%, 126.9 mg) was added. After forming a hydrogen atmosphere (hydrogen pressure: 1 atm), the mixture was stirred at 40°C for 5 hr. The catalyst was filtered off and washed with methanol (3 ml). Water (10.2 ml) was gradually added (over a time that does not decrease the temperature) to the methanol solution, and the mixture was heated to 70°C and cooled to 0°C. Crystals were collected by filtration, washed with a mixed solvent of methanol (4 ml) and water (2 ml), and dried under reduced pressure to give 1.83 g of (2R,3S)-N-(3-amino-2-hydroxy-4-phenylbutyl)-N-isobutyl-4-aminobenzenesulfonamide (yield 87%).

### <Example 10>

### Production of (2R,3S)-N-(3-amino-2-hydroxy-4-phenylbutyl)-N-isobutyl-4-aminobenzenesulfonamide (benzenesulfonamide derivative (1))

Methanol (142 ml) was added at room temperature to (2R,3S)-N-(3-benzyloxycarbonylamino-2-hydroxy-4-phenylbutyl)-N-isobutyl-4-nitrobenzenesulfonamide (14.2 g) produced in Reference Example 1 and suspended. Concentrated hydrochloric acid (35 wt%, 6.8 ml) was added and, after forming an argon atmosphere, 20% palladium hydroxide on carbon (water 51.2 wt%, 918 mg) was added. After forming a hydrogen atmosphere (hydrogen pressure: 1 atm), the mixture was stirred at 40°C for 21 hr. The catalyst was filtered off and washed with methanol (28 ml). A 6N aqueous sodium hydroxide solution (11 ml) was added to the reaction mixture and water (114 ml) was added at 4°C over 5.7 hr. The crystals were collected by filtration and washed with heptane (71 ml). The crystals were further washed twice with water (71 ml). The crystals were dried under reduced pressure to give 7.1 g of (2R,3S)-N-(3-amino-2-hydroxy-4-phenylbutyl)-N-isobutyl-4-aminobenzenesulfonamide (yield 71%). Analysis by HPLC revealed HPLC purity of 98.7% (HPLC area ratio).

### Industrial Applicability

According to the present invention, a highly pure crystal of (2R,3S)-N-(3-amino-2-hydroxy-4-phenylbutyl)-N-isobutyl-4-aminobenzenesulfonamide can be produced industrially and efficiently. Moreover, a novel crystal of (2R,3S)-N-(3-benzyloxycarbonylamino-2-hydroxy-4-phenylbutyl)-N-isobutyl-4-nitrobenzenesulfonamide and an industrially useful production method thereof can be provided.

This application is based on patent application Nos. 2001-401270 and 2002-69171 filed in Japan, the contents of which are hereby incorporated by reference.

## Claims

1. A crystal of (2R,3S)-N-(3-benzyloxycarbonylamino-2-hydroxy-4-phenylbutyl)-N-isobutyl-4-nitrobenzenesulfonamide represented by the formula (2) 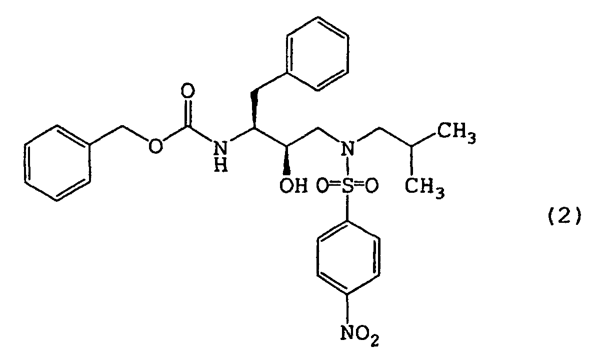 which has diffraction peaks at angles 2θ of 6.8°, 14.2° and 20.9° in a powder X-ray diffraction analysis.

2. A production method of a crystal of (2R,3S)-N-(3-benzyloxycarbonylamino-2-hydroxy-4-phenylbutyl)-N-isobutyl-4-nitrobenzenesulfonamide represented by the formula (2) 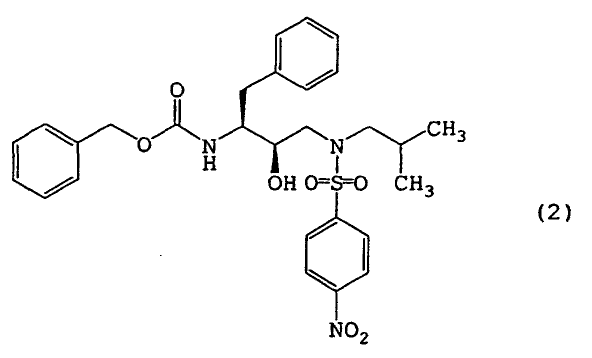 , which comprises subjecting a reaction mixture obtained by reacting (2R,3S)-3-benzyloxycarbonylamino-2-hydroxy-1-(N-isobutylamino)-4-phenylbutane with p-nitrobenzenesulfonyl chloride successively to step (i), step (ii) and step (iii), or successively to step (i), step (ii') and step (iii):
(i) a step of adding water to carry out partitioning,
(ii) a step of adding either or both of heptane and hexane to an organic layer obtained in the previous step to carry out partitioning,
(ii') a step of washing the organic layer obtained in the previous step with brine, and then adding either or both of heptane and hexane thereto, and
(iii) a step of cooling the organic layer obtained in the previous step.

3. The production method of claim 2, which comprises washing the organic layer obtained in step (i) with an aqueous sodium hydrogen carbonate solution and then subjecting the layer to step (ii) or step (ii').

4. The production method of claim 2, wherein the crystal of (2R,3S)-N-(3-benzyloxycarbonylamino-2-hydroxy-4-phenylbutyl)-N-isobutyl-4-nitrobenzenesulfonamide is the crystal of claim 1.

5. The production method of claim 2, wherein the reaction of the (2R,3S)-3-benzyloxycarbonylamino-2-hydroxy-1-(N-isobutylamino)-4-phenylbutane with p-nitrobenzenesulfonyl chloride is carried out in ethyl acetate, isopropyl acetate or a mixed solvent thereof.

6. The production method of claim 2, wherein the organic layer is cooled to a temperature lower than 20°C in step (iii).

7. A production method of (2R,3S)-N-(3-amino-2-hydroxy-4-phenylbutyl)-N-isobutyl-4-aminobenzenesulfonamide represented by the formula (1) 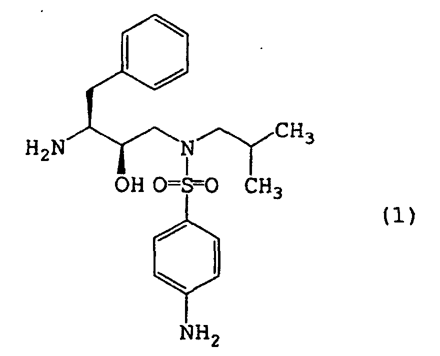 , which comprises reacting a crystal of (2R,3S)-N-(3-benzyloxycarbonylamino-2-hydroxy-4-phenylbutyl)-N-isobutyl-4-nitrobenzenesulfonamide of claim 1 with a hydrogen in the presence of a palladium catalyst.

8. The production method of (2R,3S)-N-(3-amino-2-hydroxy-4-phenylbutyl)-N-isobutyl-4-aminobenzenesulfonamide represented by the formula (1) 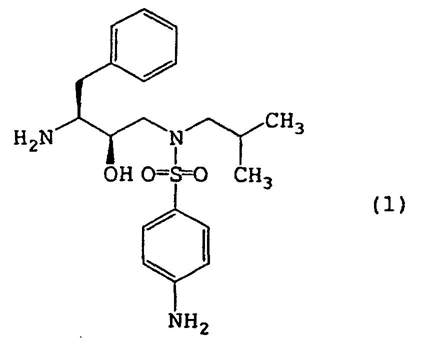 , which comprises obtaining a crystal of (2R,3S)-N-(3-benzyloxycarbonylamino-2-hydroxy-4-phenylbutyl)-N-isobutyl-4-nitrobenzenesulfonamide according to any of claims 2 to 7, and reacting the crystal with a hydrogen in the presence of a palladium catalyst.

9. The production method of claim 7 or 8, which further comprises a step of crystallizing (2R,3S)-N-(3-amino-2-hydroxy-4-phenylbutyl)-N-isobutyl-4-aminobenzenesulfonamide using a polar solvent as a good solvent and water as a poor solvent.

10. The production method of claim 7 or 8, wherein the reaction of the crystal of (2R,3S)-N-(3-benzyloxycarbonylamino-2-hydroxy-4-phenylbutyl)-N-isobutyl-4-nitrobenzenesulfonamide with a hydrogen is carried out in a polar solvent.

11. The production method of claim 9 or 10, wherein the polar solvent is alcohol or a mixed solvent of alcohol and water.

12. A production method of a crystal of (2R,3S)-N-(3-amino-2-hydroxy-4-phenylbutyl)-N-isobutyl-4-aminobenzenesulfonamide, which comprises crystallizing (2R,3S)-N-(3-amino-2-hydroxy-4-phenylbutyl)-N-isobutyl-4-aminobenzenesulfonamide represented by the formula (1) 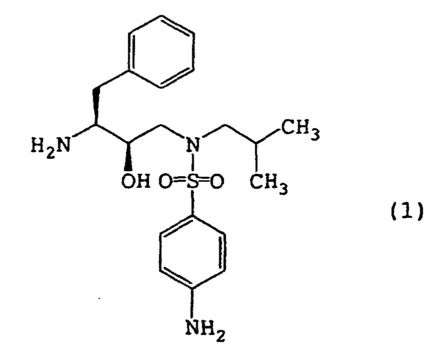 using a polar solvent as a good solvent and water as a poor solvent.

13. The production method of claim 12, wherein the (2R,3S)-N-(3-benzyloxycarbonylamino-2-hydroxy-4-phenylbutyl)-N-isobutyl-4-nitrobenzenesulfonamide represented by the formula (2) 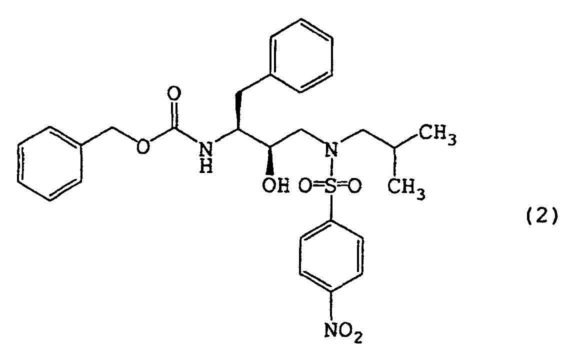 is reacted with a hydrogen in the presence of a palladium catalyst to give (2R,3S)-N-(3-amino-2-hydroxy-4-phenylbutyl)-N-isobutyl-4-aminobenzenesulfonamide represented by the formula (1), which is then crystallized using a polar solvent as a good solvent and water as a poor solvent.

14. The production method of the above-mentioned 13, wherein the (2R,3S)-N-(3-benzyloxycarbonylamino-2-hydroxy-4-phenylbutyl)-N-isobutyl-4-nitrobenzenesulfonamide represented by the formula (2) is reacted with a hydrogen in the presence of a palladium catalyst and an acid to give a salt of (2R,3S)-N-(3-amino-2-hydroxy-4-phenylbutyl)-N-isobutyl-4-aminobenzenesulfonamide represented by the formula (1), which is neutralized with alkali and then crystallized using a polar solvent as a good solvent and water as a poor solvent.

15. The production method of claim 13 or 14, wherein the reaction of (2R,3S)-N-(3-benzyloxycarbonylamino-2-hydroxy-4-phenylbutyl)-N-isobutyl-4-nitrobenzenesulfonamide with a hydrogen is carried out in a polar solvent.

16. The production method of claims 12 to 15, wherein the polar solvent is alcohol or a mixed solvent of alcohol and water.
